# EUROPÄISCHE PATENTSCHRIFT

(11) **EP 1 339 373 B1**
(45) Veröffentlichungstag und Bekanntmachung des Hinweises auf die Patenterteilung: **26.03.2008**
(21) Anmeldenummer: 01989535.8
(22) Anmeldetag: 29.11.2001
(51) Int. Cl.: A61K 6/10, A61K 49/00, C12Q 1/04

(54) **VERWENDUNG VON ABFORMMASSEN ZUR HERSTELLUNG VON BEHANDLUNGSVORRICHTUNGEN**
USE OF MOULDING COMPOUNDS FOR PRODUCING TREATMENT DEVICES
UTILISATION DE MASSES DE MOULAGE POUR FABRIQUER DES DISPOSITIFS DE MANIPULATION

(30) Priorität: 08.12.2000 DE 10061195
(43) Veröffentlichungstag der Anmeldung: 03.09.2003
(73) Patentinhaber: 3M ESPE AG, 82229 Seefeld (DE)
(72) Erfinder: FREY, Oliver, 82131 Gauting-Königswiesen (DE); BREM, Roland, 86415 Mering (DE)
(86) Internationale Anmeldenummer: PCT/EP2001/013935
(87) Internationale Veröffentlichungsnummer: WO 2002/045661

(56) Entgegenhaltungen:
- WO-A-98/45406
- DE-A- 19 753 456
- DE-A- 19 926 728
- PATENT ABSTRACTS OF JAPAN vol. 1999, no. 13, 30. November 1999 (1999-11-30) & JP 11 228597 A (TAIHO YAKUHIN KOGYO KK), 24. August 1999 (1999-08-24)

## Beschreibung

Die vorliegende Erfindung betrifft eine Behandlungsvorrichtung zum Aufbringen von Behandlungsmitteln auf infiziertes Hartgewebe, wobei die Behandlungsvorrichtung unter Verwendung einer Abformmasse, enthaltend diagnostische Zusätze, individuell herstellbar ist.

Dem Fachmann sind beispielsweise aus der EP-A-0 304 871 Single-Site-Tests bekannt, die darauf beruhen, dass von definierten Punkten im Mundraum, beispielsweise Zahnfleischtaschen, Zahnoberflächen oder Zahnwurzelkanälen einzelne Proben genommen werden. Die anschließende Analyse dieser Proben erfolgt je nach Fragestellung mit den unterschiedlichsten Methoden, wobei vier generelle Ansätze zu unterscheiden sind:
1. Die mikrobiologische Befunderhebung erfolgt häufig nach mehrtägiger Bebrütung der Proben in geeigneten Kulturmedien, weil die ursprünglich vorhandene Zahl von Mikroorganismen für eine direkte Befunderhebung nicht ausreichend ist. Nach Vermehrung der Mikroorganismen werden die Colony-Forming-Units (CFU) gezählt und auf die in der Probe befindlichen Zahl von Mikroorganismen geschlossen (Kneist, S.; Klein, C.; Rupf, S.; Eschrich, K. Quintessenz (1999) 50, 33-43). In diesen Testsystemen können sich die in der Probe befindlichen vitalen Mikroorganismen unter optimalen Bedingungen vermehren. Das Untersuchungsergebnis zeigt damit das maximal mögliche pathogene Potential des evaluierten Mikroorganismuses an, wenn sich der durch definierte Kulturmedien selektiv angezogene Mikroorganismus im Mundraum ähnlich ungehindert vermehren könnte.
   Bekanntlich liegen im Mundraum aber eben gerade nicht derartig optimale Wachstumsbedingungen vor, so dass das Testergebnis nur bedingt aussagekräftig ist.
   Darüber hinaus darf nicht übersehen werden, dass durch die Bebrütung der Proben eine Kultur pathogener Mikroorganismen angelegt wird, die mit den entsprechenden Vorsichtsmaßnahmen zur Risikominimierung in der Praxis behandelt werden müssen. Eine besondere Entsorgung ist erforderlich. Neben diesen Nachteilen sind die Bebrütungsverfahren zur mikrobiologischen Befunderhebung teuer und sehr zeitaufwendig:
2. Immunologische Methoden sind ein weiterer genereller Ansatz zur mikrobiologischen Befunderhebung in Single-Site-Tests. Hierbei werden monoklonale oder polyklonale Antikörper gegen Oberflächenstrukturen oder sezemierte Substanzen von Mikroorganismen eingesetzt. Darüber hinaus können mit entsprechenden Antikörpern beispielsweise auch Entzündungsvorgänge verfolgt werden. Beispielsweise sind hierfür WO-94/12877, US-5 665 559, WO-96/07103, WO-96/32647 zu nennen.
   Die immunologischen Methoden gemäß Absatz 2 sind im Vergleich zu den Bebrütungsverfahren gemäß Absatz 1 spezifischer, schneller und preisgünstiger, haben aber deutliche Schwächen in der Reproduzierbarkeit, die unter anderem durch die Probennahme bedingt werden. Beispielsweise befinden sich in einem Plaquebereich nicht nur vitale, sondern auch erhebliche Mengen abgestorbener Mikroorganismen. Je nach Probennahme kann das Verhältnis zwischen toten und vitalen Mikroorganismen unterschiedlich sein. Da die Antikörper nicht zwischen vitalen und toten Mikroorganismen unterscheiden können, ergibt sich eine unvorhersagbare Schwankungsbreite in der Ableitung des vorhandenen pathogenen Potentials der evaluierten Mikroorganismen (Aass, A.M.; Preus, H.R., Zambon, J.J., Gjermo, P. Scand J. Dent Res (1994) 102, 355 - 360).
3. Die Methode mit der höchsten Sensitivität beruht auf der Poly-Chain-Reaction-Technologie (PCR). Geringste Mengen Mikroorganismen können mit hoher Spezifität nachgewiesen werden. Allerdings ist die PCR-Technologie zeitaufwendig, komplex, kostenintensiv und in der Beherrschung nicht trivial (Rupf, S.; Kneist, S.; Merte,K.; Eschrich, K. Eur. J. Oral. Sci (1999) 107, 75 - 81).
4. Es wurden ferner einige Methoden beschrieben, die biochemische Marker nutzen, um Munderkrankungen zu diagnostizieren. Eine Übersicht bietet der Beitrag von J. Meyle, Deutsche Zahnärztliche Zeitschrift (1999) 54, 73-77). Die Aussagekraft der einzelnen biochemischen Marker muß differenziert unter Berücksichtigung der klinischen Studien bewertet werden und bleibt dem Fachmann vorbehalten. Hervorzuheben ist, dass die Bestimmung der biochemischen Marker mittels Single-Site-Methoden erfolgt. Beispielsweise wird auf die Patentschrift WO-98/21583 hingewiesen. Die hier benötigten Hilfswerkzeuge zeichnen sich dadurch aus, dass sie die zu untersuchenden Proben binden (WO-91/14000, EP-A-0 304 871, US-A-5 725 373). Für jede Probenstelle muß jeweils ein Hilfswerkzeug eingesetzt und individuell analysiert werden.

Prinzipiell haben alle aus dem Stand der Technik bekannten Single-Site-Methoden den entscheidenden Nachteil, dass eine näherungsweise vollständige Situationsbeschreibung im Mundraum nur mit einer hohen Zahl von Einzelproben gewonnen werden kann. Zur Probennahme werden häufig Papierspitzen verwendet, die in Zahnfleischtaschen oder Wurzelkanäle eingeführt werden (US-A-5 725 373, EP-A-0 304 871).

Es ist bekannt, dass die Parodontitisaktivität von Zahnfleischtasche zu Zahnfleischtasche in einem Patienten sehr unterschiedlich sein kann, obwohl sich die Parodontitiserreger ubiquitär in den Zahnfleischtaschen befinden. Für eine Befunderhebung müssen deshalb weit mehr als 25 Einzelproben genommen und untersucht werden, ohne sicherstellen zu können, dass nicht der eine oder andere Parodontitisherd unberücksichtigt bleibt.

Hieraus wird prinzipiell einsichtig, dass punktuelle Bestandsaufnahmen nur unbefriedigende Situationsbeschreibungen des Mundraumes zulassen. Der hohe Zeit- und Kostenaufwand der Single-Site-Techniken ist damit nur bedingt zu rechtfertigen. Single-Site-Techniken haben sich daher in der Diagnostik des Mundraumes nicht in der breiten Anwendung durchgesetzt.

Es bestand daher seit langem ein Bedürfnis, ein einfaches und kostengünstiges Verfahren zur gleichzeitigen multiplen sowie orts- und stoffspezifischen intraoralen Befunderhebung im Mundraum zur Verfügung zu haben.

Dieses Problem wird in der DE-199 26 728 angesprochen. In dieser Patentanmeldung ist ein verformbares, härtbares oder filmbildendes Trägermaterial beschrieben, das diagnostisch nutzbare Zusatzstoffe für die orts- und stoffspezifische intraorale Diagnose enthält. Es wird ausgeführt, das diese Zusatzstoffe es dem Fachmann ermöglichen, Abbildungen für den intraoral orts- und stoffspezifischen Nachweis von pathogenen Substanzen und/oder von Mikroorganismen oder zum intraoral orts- und stoffspezifischen Nachweis von Substanzen, die auf Munderkrankungen oder Heilungsprozesse hinweisen, herzustellen.

Die DE-A-198 27 417 beschreibt ein filmbildendes Material zur unterschiedlichen Modifizierung der optischen Eigenschaften unterschiedlicher Zellen mit einem Grundmaterial, wie einem Abformmaterial, und einer in diesem verteilten Modifikationssubstanz zur Verwendung in der Paradontologie. Es wird beschrieben, dass man das Modifikationsmaterial auch für Therapiezwecke einsetzen kann. Diesbezüglich wird ausgeführt, dass das Modifikationsmaterial wieder an die kranken Stellen gebracht werden soll. Die ldentifizierung der kranken Stellen erfolgt durch Fluoreszenz unmittelbar am interoralen Hartgewebe. Die Behandlung der kranken Stellen erfolgt über eine photodynamische Therapie.

Ausgehend von dem erhaltenen Befund, der unter Anwendung der in der DE-199 26 728 beschriebenen Tägermaterialien erhalten wurde, hat die weitere Behandlung der infizierten Bereiche zu erfolgen, beispielsweise dadurch, dass die infizierten Bereiche gesäubert werden und im Anschluss daran ein Behandlungsmittel aufgetragen wird.

Diese sich anschließenden Behandlungen sind verhältnismäßig zeitaufwendig und oftmals nicht hinreichend effektiv, sowie materialverschwendend, da die zu behandelnden Orte, wie in DE-A-198 27 417 beschrieben, zunächst unbekannt sind.

Folglich ist es eine Aufgabe, eine Behandlungsvorrichtung bereitzustellen, die zu eine effektivere Behandlung der infizierten Bereiche ermöglicht.

Gelöst wird diese Aufgabe durch die Verwendung von Trägermaterialien, die diagnostisch nutzbare Zusatzstoffe für die orts- und stoffspezifische Diagnose enthalten zur Herstellung einer Negativform in einem Verfahren zur Behandlung von infiziertem Hartgewebe.

Gegenstand der Erfindung ist auch die entsprechende Behandlungsvorrichtung selbst, sowie ein Verfahren zu ihrer Herstellung.

Das Vorhandensein der diagnostische Zusatzstoffe ermöglicht den intraoral orts- und stoffspezifischen Nachweis von pathogenen Substanzen und/oder von Mikroorganismen oder den intraoral orts- und stoffspezifischen Nachweis von Substanzen, die auf Munderkrankungen oder Heilungsprozesse hinweisen.

Ausgehend von dieser Information ermöglicht die vorliegende Erfindung die Bereitstellung einer Behandlungsvorrichtung zur ortsspezifischen Behandlung der infizierten Bereiche des Hartgewebes.

Unter ortsspezifisch im Sinne der Erfindung ist dabei die gezielte Behandlung der infizierten Bereiche zu verstehen, wobei vermieden werden soll - was durch Auswahl geeigneter Behandlungsmittel auch erreichbar ist -, dass nicht infiziertes Gewebe mit dem Behandlungsmittel in Kontakt kommt.

Auf diese Weise lässt sich die Menge an Behandlungsmittel auf das notwendige Minimum beschränken, wodurch Kosten eingespart werden können. Entscheidend ist aber auch, dass die Belastung des Patienten hinsichtlich Einwirkzeit und Einwirkmenge des verwendeten Behandlungsmittels drastisch reduziert werden kann.

Vorteilhaft ist außerdem, dass durch die Verwendung der Trägermaterialien nicht nur eine nahezu komplette Situationsbeschreibung der einzelnen Zähne, unter Verzicht einer großen Anzahl von Einzelproben, sowie eine Archivierung des momentanen Krankheitsbildes möglich ist sondern auch, dass die Behandlungsvorrichtung eine synchrone Behandlung des infizierten Gewebes ortsspezifisch ermöglicht. Neben okklusalen Kauflächen und vestibulären, lingualen, koronalen, apikalen, zervikalen, gingivalen, inzistalen Bereichen eines Zahnes werden durch die Zeichnungsschärfen der Trägermaterialien auch die interproximalen Bereiche zwischen den Zähnen erfasst.

Mit den Begriffen "umfassen" oder "enthalten" wird eine nicht abschließende Aufzählung von Merkmalen eingeleitet. Der Umstand, dass in den Ansprüchen das Wort "ein" vor Nennung eines Merkmals verwendet wird, schließt nicht aus, dass die genannten Merkmale mehrmals vorhanden sein können, im Sinne von "mindestens ein".

Unter dem Begriff Trägermaterial im Sinne der Erfindung ist jedes verformbare, härtbare oder filmbildende Trägermaterial, insbesondere für die intraorale Anwendung zu verstehen.

Als Trägermaterial kommen beispielsweise dentale Abformmassen oder Filme, jeweils auf Silikon-, Polyether-Silikon-, Polyether-, Alginat- oder Hydrokolloidbasis in Frage. Für manche Anwendungsbereiche, wie der Kariesdiagnose werden Alginate, bevorzugt ohne Zusatz von Phosphaten oder Pyrophosphaten verwendet. Ebenso geeignet als Trägermaterial sind alle anderen bekannten Kunststoffe, beispielsweise Polyethylene, Polypropylene, Poly(meth)acrylate, Polyurethane, Polycarbonate, Polysulfid, Polyvinylchloride oder Kautschuk. Darüber hinaus sind Hydrogele, beispielsweise auf Polyvinylpyrrolidon- oder Polyvinylalkoholbasis, als Trägermaterial geeignet. Gleichfalls geeignet für die Durchführung der erfindungsgemäßen Verfahren sind dentale Gipszubereitungen, nicht auszuhärtende plastische Massen, wie Knetmassen oder Festkörperdispersionen in Flüssigkeiten, beispielsweise Pasten und ähnliche Massen aus Silikon, Wachsen, Gelatine, Stärke, Fette und den oben genannten Trägermaterialien.

Die Basis vieler Abdruckmassen bilden additionsvernetzende oder kondensationsvernetzende Silikone, Polyether-Silikone oder Polyether. Diese Materialien sind im Stand der Technik ausführlich beschrieben worden, so dass es sich erübrigt, hier näher darauf einzugehen. Additions- oder kondensationsvernetzende Silikone sind beispielsweise in der US-A-3 897 376, in der EP-B-O 231 420 sowie in der dort auf Seite 3 erwähnten US-A-4 035 453, weiterhin in der EP-A-0 480 238 (siehe insbesondere Seite 2, Zeilen 3 - 26) und in der EP-B-O 268 347 beschrieben. Die Offenbarung dieser Schriften soll hier durch Inbezugnahme mitumfasst sein. Polyether-Silikone sind unter anderem beispielsweise in der DE-A-37 41 575 sowie in der DE-A-38 38 587 beschrieben, deren Offenbarung hier ebenfalls mitumfasst sein soll. Polyether sind beispielsweise in der DE-B-17 45 810, DE-A-43 06 997, DE-A-40 93 555, DE-C-25 15 593, DE-A-197 19 438 und US-A-34 53 242 beschrieben, deren Offenbarung hier gleichfalls mitumfasst sein soll.

Insbesondere sind Trägermaterialien auf Polyetherbasis geeignet. Hierbei umfassen die Massen beispielsweise folgende Bestandteile:
(A) 30 bis 97, bevorzugt 40 bis 89, besonders bevorzugt 45 bis 80,5 Gew.-% von mindestens einem N-Alkylaziridinopolyether mit Molmassen im Bereich von 1.000 bis 20.000 g/Mol und Aziridinoäquivalentmassen im Bereich von 500 bis 8.000 g/Äquivalent,
(B) 1 bis 10, bevorzugt 1 bis 5, besonders bevorzugt 1,5 bis 3 Gew.-% von Startersubstanzen, die geeignet sind, die Aushärtung der N-Alkylaziridinopolyether zu bewirken,
(C) 1 bis 50, bevorzugt 5 bis 45, besonders bevorzugt 8 bis 43 Gew.-% von organischen Verdünnungsmitteln,
(D) 1 bis 50, bevorzugt 5 bis 40, besonders bevorzugt 10 bis 30 Gew.-% von Modifikatoren, einschließlich Füllstoffen, Farbstoffen, Pigmenten, Thixotropiemitteln, Fließverbesserern, polymeren Eindickern, oberflächenaktiven Substanzen, Geruchsstoffen und Geschmacksstoffen,
(E) 0,0001 bis 10 Gew.-%, bevorzugt 0,01 bis 1 Gew.-% diagnostische Zusatzstoffe.

Bestandteil (A) umfasst N-Alkylaziridinopolyether, wobei die Polyether-Grundkörper Homopolymere aus Ethylenoxid, Propylenoxid oder Tetrahydrofuran, statistische Co- und Terpolymere der genannten Monomeren und bzw. oder Blockcopolymere aus Ethylenoxid und Propylenoxid sein können.

Für die Verwendung in zweikomponentigen Abformmassen sind solche Startersubstanzen gemäß Bestandteil (B) geeignet, die eine Aushärtung der gemischten Zubereitung in einem Zeitraum von 1 bis 20 Minuten zu einem elastischen Festkörper ermöglichen, wobei dieser Festkörper die Anforderungen an eine elastische Abformmasse gemäß DIN / EN 2482 erfüllt und eine Shore A-Härte (DIN 53505) von mindestens 20 nach 24 Stunden Lagerzeit besitzt.

Als Starter der Katalysatorkomponente können viele der bekannten Starter eingesetzt werden. Zweckmäßigerweise verwendet man solche Starter bzw.

Startersysteme, die eine einfache Einstellung des Aushärtungsverlaufs zulassen, keine Nebenwirkungen erzeugen und die reproduzierbare Erreichung der erforderlichen Niveaus der mechanischen Eigenschaften ermöglichen.

In der DE-C-914 325 wird die Verwendung von Oxonium-, Ammonium- und Sulfoniumsalzen als Startersubstanzen vorgeschlagen.

Eine zusammenfassende Darstellung der für die Aushärtung von N-Alkylaziridinoverbindungen verwendeten Startersubstanzen ist in O. C. DERMER, G. E. HAM, "Ethylenimine and other Aziridines" Academic Press (1969) enthalten.

Als prinzipiell geeignete Polymerisationsauslöser haben sich demnach eine große Anzahl von Verbindungsklassen und Verbindungen erwiesen. In der praktischen Anwendung der kationischen Polymerisation von Aziridinopolyethern ist es aber sehr schwierig, den gewünschten Abbindeverlauf mit ausreichend langer Verarbeitungszeit und schneller Endaushärtung einzustellen. Dieses Ziel kann durch die Verwendung von speziellen Trisalkylsulfoniumsalzen erreicht werden, wie sie beispielsweise in der EP-A-0 110 429 beschrieben sind.

Unter Verwendung von speziellen Trisalkylsulfoniumsalzen sind die Kriterien der die Härtungsgeschwindigkeit und der Eigenschaften des elastischen Festkörpers prinzipiell erreichbar.

In der Patentanmeldung DE-100 18 918 werden Starter beschrieben, die der Katalysatorkomponente einen lediglich geringen Säuregrad verleihen und die eine gut einstellbare, relativ lange Verarbeitungszeit nach erfolgter Mischung von Basiskomponente und Katalysatorkomponente ermöglichen.

Startersysteme dieses Typs sind geeignet, die Basispasten in der notwendigen Geschwindigkeit auszuhärten. Durch ihre Verwendung sind die gewünschten Eigenschaften des elastischen Festkörpers erreichbar.

Die Patentanmeldung DE-19942459 beschreibt Elastomermassen mit verbesserter Katalysatorkomponente, die sich durch eine erhöhte Dehnbarkeit auszeichnen. Gemäß dieser Erfindung werden Borsäurekomplexe als Starter eingesetzt. Diese Starter haben sich für die Aushärtung der N-Alkylaziridinopolyether besonders bewährt.

Als organisches Verdünnungsmittel entsprechend Bestandteil (C) werden Polyetherpolyole, wie Polypropylenglykole oder Mischpolyetherole mit Tetrahydrofuran- und/oder Ethylenoxid- und/oder Propylenoxid-Einheiten, Polyesterpolyole, wie Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle Ester von mehrwertigen Säuren, wie Phthalsäure oder Zitronensäure oder Ester oder Amide von Alkylsulfonsäuren und Arylsulfonsäuren verwendet.

Die Modifikatoren gemäß Bestandteil (D) sind meist feinteilige Füllstoffe, wie Alumosilikate, Fällungskieselsäuren, Quarzmehl, Wollastonit, Glimmermehl und Diatomeenerde, sowie Farbstoffe und Pigmente, deren Zusatz eine bessere Beurteilung der Mischgüte ermöglicht und die Verwechslungsgefahr vermindert, Thixotropiemittel, wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchsstoffe und Geschmacksstoffe.

Ein weiteres mögliches Trägermaterial kann auch eine polymerisierbare Flüssigkeit oder eine Lösung einer polymeren Substanz sein, die auf die zu untersuchenden Stellen aufgesprüht oder aufgetragen, beispielsweise aufgepinselt wird. Typischerweise handelt es sich hierbei um Lacke auf Nitrocellulosebasis mit einem flüchtigen Lösungsmittel sowie gegebenenfalls weiteren Hilfsstoffen, die zu einer festen Schicht aushärten, die nach Aufnahme der Markerverbindung vom Substrat abgezogen werden kann. Verwendbar sind allgemein alle Polymeren, die in geeigneten leicht flüchtigen Lösungsmitteln aufgenommen werden können. Bekannt ist beispielsweise auch die Verwendung von Polyurethanen in Aceton. Geeignete filmbildende Systeme sind aus der Farben- und Lackchemie hinreichend bekannt.

Diagnostische Zusätze sind beispielsweise, ohne dass die folgende Aufzählung limitierend für die vorliegende Erfindung zu verstehen wäre:
- Farbstoffindikatoren, beispielsweise pH-Indikatoren, wie Bromphenolblau, Kongorot, Bromkresolgrün, Oregon Green Derivate, Rhodol Derivate, Redox-Indikatoren, wie Methylenblau, 5-cyano-2,3-ditolyl tetrazolium chloride (CTC), 2-(4-iodophenyl)-3-(4-nitophenyl)-5-phenyl-2H-tetrazolium chloride (INT), 8-dimethylamino-2,3-benozophenoxazine (meldola's blue), 1-methoxy phenazine methosulphate (MPMS), 5-(3-carboxymethoxyphenyl)-2-(4,5-dimethylthiazolyl)-3-(4-sulphophenyl) teratzolium (MTS), 3-(4,5-dimethylthiazol-2-yl)-2,5 diphenyltetrazolim bromide (MTT), 3,3'-(3,3'-dimethoxy-4,4'-biphenylene)-bis [2-(4-nitrophenyl-5-phenyl)]-2H-tetrazolium chloride (NBT), Nitro-tetrazolium violet (NTV), Phenazine methosulphat (PMS), sodium 3'-[1-[(phenylamino) carbonyl]-3,4-tetrazolium]bis(4-methoxy-6-nitro) benzensulphonic acid (XTT), Phenazine ethosulfate (PES), WST-1)
- Fluoreszenzindikatoren, beispielsweise Oregon Green 488 BAPTA, Calcium green, Calcium Orange, Calcium Crimson,
- Chemolumineszenz-Indikatoren,
- Vitalitätsindikatoren, beispielsweise 5-Bromo-2' deoxyuridine,
- Andere Farbstoffindikatoren, beispielsweise p-Nitroaniline-Derivate, 2-naphthylamine-Derivate, 7-Amino-4-methylcoumarine-Derivate, 7-Amino-4-chloromethylcoumarin-Derivate, 6-Aminoquinolin-Derivate, Rhodamine-Derivate, 5,5'-dithiobis-(2-nitrobenzoic acid), Monobrombimane-Derivate, Tetramethylrhodamine-Derivate, Eosin-Derivate, Erythrosin-Derivate, Texas Red-Derivate, Coumarine-Derivate, Pyridyloxauzole-Derivate, Benzofurazan-Derivate, Naphtalene-Derivate, Didansyl Cysteine, Dansyl Derivate, Aziridine-Derivate, Pyrene-Derivate, Coomassie Blau)

Darüber hinaus können die Indikatorsubstanzen beispielsweise kovalent an Enzymen, Proteinen, Glycoproteinen, Lipopolysacchariden, Polysacchariden, polyklonale und monoklonale Antikörper, DNA, RNA Zellorganellen oder Mikroorganismen gebunden sein.

Unter diagnostischen Zusätzen werden auch Antikörper, die gegen Markerverbindungen gerichtet sind, sowie polyklonale Antikörper und deren Subklassen, sowie monoklonale Antikörper verstanden. Darüber hinaus können die Antikörper beispielsweise kovalent an Enzyme, Proteine, Glycoproteine, Lipopolysaccharide, Polysaccharide, DNA, RNA, Zellorganelle, Mikroorganismen oder andere Trägermaterialien gebunden sein.

Diagnostische Zusätze können Enzyme folgender Klassen sein, wobei die folgende Aufzählung beispielsweise und nicht limitierend für die Erfindung ist:
- Oxidoreductasen und deren Unterklassen, beispielsweise Dehydrogenasen, wie Lactatdehydrogenase, Oxidasen, Peroxidasen, Reductasen, Monooxygenasen, Dioxygenasen;
- Transferasen und deren Unterklassen, beispielsweise C₁-Transferasen, Glycosyl-Transferasen, wie Glucosyltransferasen, Fructosyltransferasen, Aminotransferasen, Phospho-Transferasen;
- Hydrolasen und deren Unterklassen, beispielsweise Esterasen, Glycosidasen, wie Glucanase, Fructanase, Peptidasen, beispielsweise Dipeptidylpeptidasen Arg-Gingipain, Lys-Gingipain, Collagenasen, Gelatinasen, Cathepsine, Elastase, Amidasen,
- Lyasen und deren Unterklassen, beispielsweise C-C-Lyasen, C-O-Lyasen, C-N-Lyasen, C-S-Lyasen;
- lsomerasen und deren Unterklassen, beispielsweise Epimerasen, cis-trans-losmerasen, intramolekulare Transferasen;
- Ligasen und deren Unterklassen, beispielsweise C-C-Ligasen, C-O-Ligasen, C-N-Ligasen, C-S-Ligasen.

Man kennt heute über 2000 verschiedene Enzyme. Zu ihrer Klassifizierung wurde ein System entwickelt, das Wirkungs- und Substratspezifität berücksichtigt. Daraus ergibt sich, dass zu jedem Enzym spezifische Substrate und/oder Coenzyme (NAD(P), NAD(P)H, FAD, FMN, Liponamid, Ubichinon, Häm, ATP, ADP, AMP, GTP, GDP, GMP, UTP, UDP, UMP, CTP, CDP, CMP, Coenzym A, Thiamindiphosphät, Pyridoxalphosphat, Biotin, Tetrahydrofolat gehören. Diese spezifischen Substrate und/oder Coenzyme müssen ais diagnostischer Zusatz vorhanden sein, wenn beispielsweise ein oder mehrere Enzyme als Markersubstanz dienen. Umgekehrt gilt natürlich, dass spezifische Enzyme als diagnostische Zusätze verwendet werden können, wenn spezifische Substrate beispielsweise Zuckerphosphate, Milchsäure/Lactat, Pyruvat, Essigsäure/Acetat, Propionsäure/Propionat, Ameisensäure/Formiat, Peptide, synthetische Peptide als Markersubstanzen dienen.

Darüber hinaus können die Enzyme kovalent an Trägermaterial gebunden sein.

Diagnostische Zusätze können auch solche Substanzen sein, die begleitend vorliegen müssen, um die Markersubstanzen diagnostizieren zu können. Solche Substanzen umfassen:

Puffersubstanzen, beispielsweise Natriumphosphat, Natriumhydrogenphosphat, Natriumdihydrogenphophat, Kaliumphosphat, Kaliumhydrogenphosphat, Kaliumdihydrogenphosphat, Natriumpyrophosphat, Natriumcarbonat, Kaliumcarbonat, Natriumhydrogencarbonat, Kaliumhydrogericarbonat, Natriumtetraborat, Essigsäure/Acetat, Citronensäure/Citrat, Diethylbarbitursäure, Tris(hydroxymethyl)-aminomethan (TRIS), Glycin, Glycylglycin, N-(2-Acetamido)-2-aminoethansulfonsäure (ACES), N-(2-Acetamido)iminodiacetat(ADA), N,N-Bis(2-hydroxyethyl)-2-aminoethansulfonsäure (BES), N,N-Bis(2-hydroxyethyl)glycin (BICINE), 2,2-Bis-(hydroxyethyl)-iminotris(hydroxymethyl)methan (BIS-TRIS), 2-(Cyclohexylamino)ethansulfonsäure (CHES), 2-[4-(2-Hydroxyethyl-1-piperazin)]-ethansulfonsäure (HEPES), 3-[4-(2-Hydroxyethyl-1-piperazinyl)]propansulfonsäure (HEPPS), 2-Morpholinoethansulfonsäure (MES), 3-Morpholinopropansulfonsäure (MOPS), Piperazin-1,4-bis(2-ethansulfonsäure) (PIPES), N-[Tris(hydroxymethyl)-methyl]-2-aminoethansulfonsäure (TES), N-[Tris(hydroxymethyl)-methyl]-glycin (TRICINE);
- Säuren, beispielsweise Schwefelsäure, schweflige Säure, Phosphorsäure, Salzsäure, Essigsäure, Salpetersäure;
- e Basen, beispielsweise Natriumhydroxid, Kaliumhydroxid, Lithiumhydroxid, Ammoniak, Calciumhydroxid, Magnesiumoxid;
- Lösungsmittel, beispielsweise Wasser, Methanol, Ethanol, Isopropanol, Propanol, Glycerin, Dimethylsulfoxid, Tetrahydrofuran, Aceton, Butanon, Cyclohexan, Toluol, Methylenchlorid, Chloroform, Alkane, Essigsäureethylester;
- Salze, beispielsweise Magnesiumchlorid, Magnesiumsulfat, Magnesiumnitrat, Calciumchlorid, Calciumsulfat, Calciumnitrat, Eisen(III)chlorid, Eisen(II)chlorid, Zinkchlorid, Zinksulfat, Nickeichlorid, Manganchlorid, Ammoniumsulfat, Natriumsulfat, Natriumchlorid, Kaliumchlorid, Natriumphosphate, Kaliumphosphate;
- e andere Substanzen, beispielsweise Glutathion, Bovine Serum Albumin, Saccharose, Glucose, Fructose, Trehalose, Polyethylenglycole, Polyvinylpyrrolidone, Wasserstoffperoxid.

In einer speziellen Ausführungsform der Erfindung können die diagnostischen Zusätze in mikroverkapselter Form vorliegen. In einer Mikrokapsel kann eine Vielzahl von Molekülen diagnostischer Zusatzstoffe eingeschlossen sein. Von besonderem Vorteil ist bei der Verwendung von mikroverkapselten diagnostischen Substanzen der auftretende Potenzierungseffekt.

Ganz allgemein können bei der Verwendung mehrkomponentiger Diagnosesysteme gemäß der Erfindung, also Systeme, bei denen die notwendigen Bestandteile zum Nachweis in mehreren Komponenten gelagert werden, die einzelnen Komponenten getrennt voneinander, jedoch jeweils eingeschlossen in Mikrokapseln, oder auch teilweise mikroverkapselt und teilweise frei vorliegen. Selbstverständlich ist es auch möglich, bei mehr als zweikomponentigen Diagnosesystemen, mindestens zwei Komponenten jeweils mikroverkapselt und mindestens eine andere Komponente frei im Trägermaterial vorrätig zu halten. Essentiell ist jeweils nur, dass eine Reaktion der diagnostischen Zusätze zum gewünschten Endprodukt durch das getrennte Vorhalten der einzelnen Komponenten solange unterbunden wird, bis ein Reaktionspartner durch eine Zerstörung der Mikrokapselwand freigesetzt wird.

Da Abformmaterialien üblicherweise zweikomponentig angeboten werden, kann es vorteilhaft sein, verschiedene Komponenten der Wirkstoffe in verschiedenen Komponenten der Abformmassen, namentlich der Basis- und der Katalysatorpaste, mikroverkapselt oder frei vorzuhalten.

Bei der Auswahl von geeigneten Trägermaterialien ist allgemein darauf zu achten, dass diese mit den diagnostischen Substanzen kompatibei sind. Beispielsweise sollte bei der Verwendung von Fluoreszenzfarbstoffen die Trägermaterialien selbstverständlich keine Bestandteile enthalten, die im relevanten Wellenlängenbereich selbst fluoreszieren. Die Forderung nach inerten Trägermaterialien im Sinne der diagnostischen Zielsetzung ist für den Fachmann trivial und kann vom Fachmann problemlos beachtet werden.

Die verwendeten diagnostisch nutzbaren Zusatzstoffe sind zum Teil kommerziell erhältlich und können gegebenenfalls physikalisch, chemisch, biochemisch oder gentechnologisch verändert werden, wobei dies insbesondere für Enzyme und deren Substrate, für Antikörper und deren Antigene und für Oligonukleotide und Polynukleotide gilt.

Unter dem Begriff identifizierbares Signal im Sinne der Erfindung ist jedes detektierbares Signal zu verstehen. Hierunter fallen beispielsweise Farbsignale, beispielsweise fluoreszierende, UV-, VIS-, phosphoreszierende oder lumineszierende Signale, die gegebenenfalls mit speziellen Geräten detektiert werden müssen. Ebenso können Signale durch Anwendung der erfindungsgemäßen Verfahren erzeugt werden, die durch Thermographie, Spektroskopie, Chromatographie oder auch durch Analyse der Topographieänderung der Trägermaterialien wahrgenommen werden können. Bevorzugt sind durch das menschliche Auge optisch erfassbare Signale.

Unter dem Begriff Negativform im Sinne der Erfindung ist die Form zu verstehen, die erhalten wird, wenn die ausgehärtete Abformmasse vom Hartgewebe oder einem entsprechenden Modell des Hartgewebes abgehoben wird. Hierbei kann es sich um den Abdruck eines gesamten Ober- oder Unterkiefers handeln oder auch nur um einzelne Bereiche davon. Umfasst sind auch die Negativformen, die unter zwischenzeitlicher Anfertigung einer Positvform unter Verwendung der zunächst angefertigten Negativform hergestellt wurden.

Unter den nachzuweisenden pathogenen Substanzen und/oder Mikroorganismen oder Substanzen, die auf Munderkrankungen oder Heilungsprozessen hinweisen bzw. zur Bildung von infiziertem Hartgewebe im Sinne der Erfindung führen oder beitragen sind beispielsweise genannt:
1. Stoffwechseiprodukte von Bakterien, Viren oder Pilzen, beispielsweise Antigene, Lipide, Proteine, Peptide, Polysaccharide, DNA, RNA, Zucker, Aminosäuren, Carbonsäuren, beispielsweise Milchsäure und Propionsäure, sowie andere niedermolekulare, anionische, kationische oder neutrale Substanzen sowie deren Kombinationen, die sich beispielsweise aus ionischen, polaren, unpolaren, hydrophoben, kovalenten oder adhäsiven Wechselwirkungen ergeben.
2. Oberflächenstrukturen von Bakterien, Viren oder Pilzen, bestehend beispielsweise aus Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
3. Humane bzw. tierische Substanzen, die als Antwort auf Infektionen durch Bakterien, Viren oder Pilze gebildet werden, bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
4. Humane bzw. tierische Substanzen, die auf Munderkrankungen hinweisen, die nicht *a priori* auf eine Infektion durch Bakterien, Viren oder Pilze beruhen (beispielsweise Krebserkrankungen), bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
5. Substanzen, die sich in Strukturen befinden, die als die Folge von oder die Voraussetzung für die Entstehung von Munderkrankungen, beispielsweise Plaque oder Biofilm, bekannt sind, bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.
6. Substanzen die auf laufende Heilungsprozesse hinweisen, die als die Folge von Munderkrankungen oder Verletzungen, beispielsweise Gewebe und/oder Knochenregeneration, bekannt sind, bestehend beispielsweise aus Antikörpern, Antigenen, Lipiden, Proteinen, Peptiden, Polysacchariden, DNA, RNA, Zuckern, Aminosäuren oder anderen niedermolekularen, anionischen, kationischen oder neutralen Substanzen sowie deren Kombinationen, die sich beispielsweise aus ionischen, polaren, unpolaren, hydrophoben, kovalenten oder adhäsiven Wechselwirkungen ergeben.

Zu den diagnostizierbaren und behandelbaren Munderkrankungen gehören auch Karies, Early Onset Parodontitis, prepubertale Parodontitis, juvenile Parodontitis, schnell verlaufende progressive Parodontitis (RPP), adulte Parodontitis, refraktäre Parodontitis, Gingivitis, Halitosis, Infektionen mit Candida albicans, Candida krusei, Candida glabrata, Candida lusitaniae, Candida dubliniensis, Krebs.

Die verschiedenen Formen der Parodontitis, die mit der erfindungsgemäßen Behandlungsvorrichtung behandelt werden können, sind kausal mit der Infektion durch Actinobacillus actinomycetemcomitans, Bacterioides forsythus, Campylobacter rectus, Capnocytophage ochracea, Capnocytophage gingivalis, Eikenella corrodens, Fusobacterium nucleatum, Porphyromonas asaccharolytikus, Porphyromonas gingivalis, Prevotella dentalis, Prevotella intermedia, Prevotella nigrescens, Treponema denticola verbunden. Durch die Verwendung der Abformmassen lassen sich Gegenwart und Menge der Bakterien in der Sulkusflüssigkeit bestimmen. Hierfür eignen sich spezifische polyklonale Antikörper und deren Subklassen oder monoklonale Antikörper, die gegen Oberflächenantigene dieser Bakterien, beispielsweise Fimbriae, extrazelluläre Polysaccharide, Adhesine gerichtet sind.

Karies ist kausal mit der. Infektion durch Streptococcus salivarius salivarius, Streptococcus vestibularis, Streptococcus thermophilus, Streptococcus mutans, Streptococcus rattus, Streptococcus sobrinus, Streptococcus cricetus, Streptococcus downei, Streptococcus macacae, Streptococcus ferus, Streptococcus milleri, Streptococcus anginosus, Streptococcus constellatus, Streptococcus intermedius, Streptococcus mitis, Streptococcus oralis, Streptococcus sanguis, Streptococcus gordonii, Strepto-coccus parasanguis, Streptococcus crista, Streptococcus mitior, Lactobacillus acidophilus, Lactobacillus alimentarius, Lactobacillus brevis, Lactobacillus buchneri, Lactobacillus casei, Lactobacillus paracasei ss paracasei, Lactobacillus paracasei ss rhamnosus, Lactobacillus paracasei ss tolerans, Lactobacillus delbrueckii, Lactobacillus delbrueckii ss lactis, Lacto-bacillus delbrueckii ss delbrueckii, , Lactobacillus delbrueckii ss bulgaricus, Lactobacillus endocarditis, Lactobacillus fermentum, Lactobacillus gasseri, Lactobacillus pseudoplantarum, Lactobacillus rhamnosus, Lactobacillus salivarius, Actinomyces israelii, Actinomyces odontolyticus, Actinomyces actinomycetemcomitans, Eikenella, Branhamella catarrhalis, Veillonella alcalescens, Veillonella parvula, Actinomyces naeslundii, Rothia dentocariosa, verbunden. Durch die Verwendung der Abformmassen können mit polyklonalen Antikörpern und deren Subklassen oder monoklonalen Antikörpern, die gegen die verschiedenen Oberflächenantigene dieser Bakterien, beispielsweise Proteine, Lipopolysaccharide, Glycoproteine, Fimbriae, extrazelluläre Pollysaccharide, Adhesine, Lipoteichonsäurederivate, Glucan-Bindungsproteine, Collagen-Bindungsproteine gerichtet sind, Gegenwart und Menge der kariogenen Bakterien diagnostiziert werden und darauf aufbauend ein geeignetes Behandlungsmittel auf die Negativform aufgebracht werden.

Unter dem Begriff Behandlungsmittel im Sinne der Erfindung sind grundsätzlich alle Mittel zu verstehen, die geeignet sind, die unter Verwendung der Abformmasse, enthaltend diagnostische Zusätze, mit diesen Zusätzen detektierten Infektionen zu behandeln.

Geeignete Behandlungsmittel umfassen: Fluoridierungsmittel, wie Fluoridierungslacke oder -gele, Antibiotika, Bakteriostatika bzw. Bakteriozide, wie Chlorhexidin in Form von Lacken oder Gelen sowie Triclosan, quartäre Ammoniumverbindungen und Mineralienmischungen, welche eine effiziente Remineralisierung der Zahnhartsubstanz ermöglichen.

Unter dem Begriff "vorbehandelt" im Sinne der Erfindung ist jegliche Art und Weise einer Modifizierung der Oberfläche zu verstehen. Insbesondere fallen hierunter mechanische Vorbehandlungen, beispielsweise durch teilweises Abtragen der Oberfläche oder Anrauben. Umfasst ist aber auch das Aufbringen einer chemischen Substanz oder eines Substanzgemisches.

Das Abtragen kann erfolgen unter Verwendung von Skalpellen, Scheren oder sonstigen, dem zahnärztlichen Fachpersonal bekannten, Schneidevorrichtungen. Die erfindungsgemäße Behandlungsvorrichtung umfasst üblicherweise einen Abformlöffel und eine darin befindliche Negativform aus einem Trägermaterial, enthaltend diagnostische Zusätze. Auf diese Negativform ist an mindestens einer Stelle ein Behandlungsmittel aufgebracht.

Diese Behandlungsvorrichtung wird anschließend für eine bestimmte Zeit wieder auf das ursprünglich abgeformte Hartgewebe aufgesetzt.

Bedingt durch die Art und Weise wie und wo das Behandlungsmittel auf die Negativform aufgebracht wurde, befindet sich nun dieses Behandlungsmittel ortsspezifisch an den infizierten Stellen des Hartgewebes.

Die erfindungsgemäße Behandlungsvorrichtung ermöglicht damit einen effektiven Einsatz von Behandlungsmitteln. Effektiv zum einen deshalb, weil nur die erforderliche Menge an Behandlungsmittel aufgebracht wird, effektiv zum anderen deshalb, weil das Behandlungsmittel nur an den Stellen aufgebracht wird, wo eine Behandlung erforderlich ist.

Die Erfindung weist weiterhin folgende Vorteile auf: Der Behandler kann die Therapierung der erkrankten bzw. infizierten Zahnhartsubstanzbereiche schneller als bislang durchführen, weil er mit der vorliegenden Erfindung mehrere betroffene Stellen zeitgleich versorgen kann und nicht wie bisher eine nach der anderen. Dadurch werden auch die Belastung für den Patienten minimiert und letzlich Behandlungskosten eingespart.

Durch die Verwendung der Trägermaterialien können in der Sulkusflüssigkeit Enzymaktivitäten gemessen werden, die einen Hinweis auf die Gegenwart und metabolische Aktivität eines Bakteriums oder einer Gruppe der genannten Bakterien ergeben. Die Trypsin-ähnliche Protease-Aktivität, bevorzugt die Dipeptidylpeptidase-Aktivität, besonders bevorzugt Arg-Gingipain-Aktivität und Lys-Gingipain-Aktivität wird diagnostisch genutzt. Zur Bestimmung der Arg-Gingipain-Aktivität können synthetische Peptide eingesetzt werden, die mindestens ein Arg-Rest (in P1-Position) neben der detektierbaren Abgangsgruppe enthalten. Zur Bestimmung der Lys-Gingipain-Aktivität können synthetische Peptide eingesetzt werden, die mindestens ein Lys-Rest (in P1-Position) neben der detektierbaren Abgangsgruppe enthalten. Neben p-Nitroanilin-Derivaten, beispielsweise Nα-Benzoyl-DL-arginin-p-nitroanilid, 2-Naphtylamin-Peptidderviaten, beispielsweise Nα-Benzoyl-DL-arginin-β-naphtylamid können 6-Aminoquinolin-Peptidderivate, Rhodamin-Peptidderivate sowie Cumarin-Peptidderivat, beispielsweise 7-Amido-4-methylcumarin, wie N-t-Boc-Val-Pro-Arg-7-Amido-4-methylcumarin und 7-Amino-4-chloromethylcumarin, wie N-t-Boc-Val-Pro-Arg-7-Amido-4-chloromethylcumarin als detektierbare Abgangsgruppen eingesetzt werden.

Durch die Verwendung der Trägermaterialien lassen sich mit polyklonalen Antikörpern und deren Subklassen oder monoklonalen Antikörpern die bakteriellen Substanzen diagnostizieren, die zur Induktion von Zytokinen führen. Bevorzugt werden Antikörper gegen Lipopolysaccharide, Lipoarabinomannan, Peptidoglycane, Teichonsäurederivate, extrazelluläre Polysaccharide und Lipid A. Durch die Verwendung der Trägermaterialien kann die durch Parodontitiserreger induzierte Zytokininbildung mit polyklonalen Antikörpern und deren Subklassen oder monoklonalen Antikörpern diagnostiziert werden. Antikörper gegen die Interleukine IL-1, IL-2, IL-3, IL-4, IL-5, IL-6, IL-7, IL-8, Tumornecrosisfaktor TNFα, Interferone α,β,γ, Colony-forming Faktoren M-CSF, Wachstumsfaktoren EGF, TGFα und Chemokine MCP können eingesetzt werden.

Durch die Verwendung der Trägermaterialien kann die Zerstörung parodontalen Gewebes über die Enzymaktivitäten von alkalischer Phosphatase, Arylsulfatase, Aspartataminotransferase, β-Glucuronidase, Cathepsine (G,B,D), Elastase, Hyaluronidase, Lactatdehydrogenase, Lysozym, Matrixmetalloproteinasen (Kollagenasen, Gelatinasen), Tissue Inhibitors Metalloproteinases (TIMP), Stromelysin, Lactoferrin, Tryptase und Myeloperoxidase diagnostiziert werden.

Durch die Verwendung der Trägermaterialien können mit polyklonalen Antikörpern und deren Subklassen oder monoklonalen Antikörpern die molekularen Marker für Gingivitis diagnostiziert werden. Zu diesen gehören Zytokine, beispielsweise Interleukine IL-1, IL-2, IL-4, IL-6, TNFα und Arachidonsäurederviate, beispielsweise Prostaglandin E₂.

Durch die Verwendung der Trägermaterialien können extrazelluläre Enzymaktivitäten kariogener Bakterien diagnostiziert werden, beispielsweise Proteasen, bevorzugt Glucosyltransferasen, Glucanase, Fructosyltransferase, Fructanase.

Durch die Verwendung der Trägermaterialien können Stoffwechselprodukte kariogener Bakterien diagnostiziert werden, beispielswesie Buttersäure, Ameisensäure, bevorzugt Essigsäure, Propionsäure, besonders bevorzugt Milchsäure. Die mit der Säurefreisetzung einhergehende Versauerung des umgebenden Milieus kann darüber hinaus mit pH-lndikatoren, beispielsweise mit Bromphenolblau, Kongorot, Bromkresolblau, bevorzugt Rhodolderivate, besonders bevorzugt Oregon Green-Derivate nachgewiesen werden. Als Folge der Versauerung des pHs im umgebenden Milieu, wie Plaque werden aus der Zahnhartsubstanz Calciumionen herausgelöst. Durch die Verwendung der Abformmasse kann dieser Prozess mit Calciumindikatoren, beispielsweise Calcium Crimson, bevorzugt Calcium Green, Calcium Orange, besonders bevorzugt Calcium Oregon Green 488 BAPTA diagnostiziert werden.

Durch die Verwendung der Trägermaterialien kann die Zunahme oder die Abnahme der oben genannten Markerverbindungen als Maß für den Heilungsprozess herangezogen werden.

Überraschend ist, dass trotz der ablaufenden dynamischen Prozesse in der Mundhöhle, die einem ständigen Flüssigkeitsaustausch durch die Sekrete der Speicheldrüsen und der Sulkusflüssigkeit unterliegt, ausreichend hohe Konzentrationen von Marker-Verbindungen auf den Oberflächen der erfindungsgemäßen Trägermaterialien oder in den Trägermaterialien erhalten werden, die es gestatten, eine sichere Diagnose auch im Rahmen von Routinebehandlungen zu realisieren.

Vorteilhaft ist es, dass durch die Verwendung der Abformmasse eine nahezu komplette Situationsbeschreibung des Mundraumes, unter Verzicht einer großen Anzahl von Einzelproben, sowie eine Archivierung des momentanen Krankheitsbildes möglich ist. Hierbei ist besonders die Verwendung von additionsvernetzenden Silikonabformmaterialien von Interesse, da die Abdrücke praktisch unbegrenzt haltbar sind.

Das Trägermaterial enthält in einer bevorzugten Ausführungsform mindestens eine Komponente oder aber zur Vereinfachung der diagnostischen Prozedur alle benötigten Komponenten des diagnostischen Testsystems. Diese diagnostischen Zusätze können beispielsweise über ionische, polare, unpolare oder hydrophobe Wechselwirkungen auf bzw. im Trägermaterial örtlich fixiert werden. Eine örtliche Fixierung von diagnostischen Zusätzen ist auch dadurch möglich, dass die diagnostischen Zusätze zuerst an hochmolekulare Träger fixiert und anschließend in die Trägermasse eingeknetet werden. Hierdurch wird die Diffusionsbewegung der diagnostischen Zusätze im Trägermaterial kontrolliert. Die Ausbildung von Mikrostrukturen und/oder Mikroräumen in den Trägermaterialien beispielsweise in Form von Schäumen kann die Aufnahme und

Fixierung der Komponenten unterstützen. Die Komponenten können in den erfindungsgemäßen Trägermaterialien frei verfügbar oder in einer anderen Phase vorliegen.

Die Trägermaterialien enthalten 0,0001 bis 10 Gew.-%, bevorzugt 0,01 bis 1 Gew.-% diagnostische Zusätze, jedoch mindestens soviel Zusätze, dass die gewünschte Wirkung wahrgenommen werden kann. Bei der Anwendung des erfindungsgemäßen Verfahrens müssen diagnostische Zusätze in einer solchen Menge auf Trägermaterialien aufgebracht werden, dass die gewünschte Wirkung wahrgenommen werden kann.

Folgende Kombinationen von Trägermaterial, diagnostischen Zusätzen und Behandlungsmittel haben sich besonders bewährt:

Als Trägermaterial werden z.B. bevorzugt Alginatabformmassen eingesetzt welche mit diagnostischen Zusätzen versehen sind, die eine ortsspezifische Bestimmung von kariösen Prozessen durch ein Farbsignal ermöglichen. Derartige Trägermaterialien können an den angefärbten Stellen in hervorragender Weise mit Fluoridgelen, wie z.B. Elmex-Gelee (Fa. Wybert) versehen werden mit dem Ziel der punktgenauen Tiefenfluoridierung.

Eine weitere bevorzugte Kombination stellen Alginat- oder Polyetherabformmassen dar, welche diagnostischen Zusätze enthalten, die eine ortsspezifische Anfärbung von parodontal infizierten Zahnfleischtaschen ermöglichen. Diese Trägermaterialien können sehr gut an den angefärbten Stellen mit lokal wirksamen Antibiotika in Form von Lacken versehen werden.

Die Behandlungsvorrichtung lässt sich beispielsweise dadurch herstellen, dass man ein Trägermaterial, enthaltend diagnostische Zusätze, in einen Abformlöffel füllt und mit diesem einen Negativabdruck von Hartgewebe nimmt. Infizierte Stellen des Hartgewebes erzeugen auf der dem Hartgewebe zugewandten Oberfläche des Trägermaterials identifizierbare Signale.

An diesen Stellen wird anschließend ein Behandlungsmittel aufgebracht.

In einer anderen Ausführungsform wird unter Verwendung der Negativform ein Postivabdruck erzeugt, der seinerseits als Vorlage verwendet wird, um eine individuelle dauerhaft verwendbare Behandlungsvorrichtung, vorzugsweise in Form einer Bissschiene, herzustellen.

Zur Anfertigung einer solchen Bissschiene eignen sich die dem Fachmann zur Herstellung von individuellen Bissschienen oder Abformlöffeln bekannten Materialien.

Eine solche individuelle Behandlungsvorrichtung weist üblicherweise einen deutlich verbesserten Tragekomfort auf und lässt sich wiederholt unabhängig von einem Besuch beim Arzt anwenden.

Auf der Oberfläche der individuellen Bissschiene werden die Bereiche gekennzeichnet, die in der Negativform als infizierte Bereiche auf dem Hartgewebe detektiert wurden. Auf diese Bereiche kann dann wiederholt ein Behandlungsmittel aufgebracht werden.

Dies ist insbesondere dann von Vorteil, wenn die Behandlung der infizierten Stellen des Hartgewebes über einen längeren Zeitraum von beispielsweise mehreren Wochen notwendig erscheint.

In einer bevorzugten Ausführungsform wird die Oberfläche des Trägermaterials an diesen Stellen vorbehandelt, beispielsweise in der Form, dass an diesen Stellen Material entfernt wird, um ein Reservoir für das aufzubringende Behandlungsmittel zu schaffen:

In Abhängigkeit von aufzubringendem Behandlungsmittel kann es auch vorteilhaft sein, wenn vor dem Aufbringen eine Haftgrundlage geschaffen wird.

Eine solche Haftgrundlage kann beispielsweise durch Aufrauhen der Oberfläche in diesem Bereich geschaffen werden. Zusätzlich oder alternativ kann auch eine Primer-artige Substanz aufgetragen werden.

### Die Erfindung wird nachstehend näher an Hand von Beispielen beschrieben:

### Anwendungsbeispiel 1

### Nachweis von Arg-Gingipain über eine Polyetherabformmasse

In einem laborüblichen Dreifingerkneter wurde eine Basispaste hergestellt, indem bis zur Homogenität 53,2 Gewichtsteile eines Aziridinopolyethers, der gemäß Beispiel 12 der DE-PS-17 45 810 erhalten wurde, mit 18,1 g eines hydrierten Palmöls und 6,4 Gewichtsteilen Dibenzyltoluol vermischt wurden. Diese Masse wurde mit 11,8 Teilen eines Copolymers aus Ethylenoxid- und Tetramethylenoxideinheiten einer mittleren molaren Masse von 6500, sowie 0,1 Teilen Laurylimidazol und 5,0 Teilen eines Blockcopolymers aus Ethylenoxid- und Propylenoxideinheiten mit einer mittleren Molmasse von 3500 vereinigt. Diese Masse wurde anschließend mit 5,3 Gewichtsteilen Kieselgur vermischt.

Eine Katalysatorpaste wurde durch Homogenisieren von 33,8 Gewichtsteilen Acetyltributylcitrat mit 14,1 Teilen Ethylenoxid-Propylenoxid-Blockcopolymer und 19,0 Teilen eines Sulfoniumsalzes vermischt, das gemäß Beispiel 31 der DE-PS-25 15 593 erhalten wurde. Diese Masse wurde vereinigt mit 11 Teilen Kieselgur und 20,5 Teilen pyrogener Kieselsäure sowie 1 Teil Titandioxid. Anschließend wurden als Puffersubstanzen 0,7 g Tris-(hydroxymethyl)aminomethan, 0,8 g Glycylglycin und als Substrat 200 µg N-t-Boc-Val-Pro-Arg-7-Amido-4-methylcumarin zugegeben.

Basis- und Katalysatorpaste wurden im Volumenverhältnis 5:1 vermischt und härteten nach ca. 8 Minuten zu einem homogenen Gummi aus. Eine Dotierung der Oberfläche dieses Gummis während der Abbindephase mit 2 µl Arg-Gingipain-haltiger Lösung (Stammlösung: 0,5 mg/ml Arg-Gingipain in 200 mM Tris-(hydroxymethyl)aminomethan pH 7,6) ergab nach wenigen Minuten an dieser Stelle eine intensiv blaue Fluoreszenzemission bei einer Anregungswellenlänge von 360 nm.

Im Anschluss daran werden die markierten Stellen mit Elyzol Dentalgel (Fa. Colgate, Wirkstoff Metronidazolbenzoat) eingepinselt.

### Anwendungsbeispiel 2

### Nachweis von Arg-Gingipain auf Alginatprüfkörpern

Zu 10 g Alginat (Palgat Plus Quick, Fa. ESPE Dental AG) wurden 20 ml Lösung, enthaltend 0,12 g Tris-(Hydroxymethyl)aminomethan, 100 µg N-t-Boc-Val-Pro-Arg-7-Amido-4-methylcumarin, pH 7,6 gegeben und mit einem breiten Kunstoffspatel innerhalb 1 min zu einer homogenen Masse geknetet. Während der Abbindephase wurde der Alginatprüfkörper mit 2 µl Arg-Gingipain-haltiger Lösung (Stammlösung: 0,5 mg/ml Arg-Gingipain in 200 mM Tris-(hydroxymethyl)aminomethan pH 7,6) dotiert. An dieser Stelle konnte nach 5 min eine intensiv blaue Fluoreszenzemission bei einer Anregungswellenlänge von 360 nm beobachtet werden.

Im Anschluss daran werden die markierten Stellen mit Elyzol Dentalgel (Fa. Colgate, Wirkstoff Metronidazolbenzoat) eingepinselt.

### Anwendungsbeispiel 3

### Nachweis von Arg-Gingipain über eine Alginatabformmasse in Zahnfleischtaschen

Zu 20 g Alginat (Palgat Plus Quick, Fa. ESPE Dental AG) wurden 40 ml Lösung, enthaltend 0,24 g Tris-(Hydroxymethyl)aminomethan, 0,26 g Glycylglycin, 200 µg N-t-Boc-Val-Pro-Arg-7-Amido-4-methylcumarin gegeben und mit einem breitem Kunstoffspatel innerhalb 1 min zu einer homogenen Masse geknetet. Die Alginatmasse wurde in einen handelsüblichen Abformlöffel eingebracht und am Ober- oder Unterkiefer eines Parodontitis-Patienten für 5 min plaziert. Intensiv blaue Fluoreszenzemissionen konnte bei einer Anregungswellenlänge von 360 nm an einzelnen Zahnfleischtaschenrändem beobachtet werden. Im Anschluss daran werden die markierten Stellen mit Elyzol Dentalgel (Fa. Colgate, Wirkstoff Metronidazolbenzoat) eingepinselt.

Die so präparierte ausgehärtete Abformmasse wird nun im Munde des Patienten reponiert und dort für einen Zeitraum von ca. 10 Minuten belassen.

### Anwendungsbeispiel 4

### Nachweis von Milchsäure auf Alginatprüfkörpern

Zu 5 g Alginat wurden 10 ml Lösung, enthaltend 0,065 g Glycylglycin, 0,06 g Tri-(hydroxymethyl)aminomethan, 9 mg NAD, 0,23 mg Phenazinmethosulfat, 0,75 mg 3-(4,5-Dimethylthiazolyl-2)-2,5-diphenyltetrazoliumbromid (MTT), 463 Units Lactatdehydrogenase aus Schweineherz gegeben und mit einem breitem Spatel innerhalb 1 min zu einer homogenen Masse geknetet. Der Alginatprüfkörper wurde mit 5 µl einer 10 mM Calactat Lösung in 100 mM Tris-(hydroxymethy)aminomethan pH 9,0 dotiert. Nach 4 min war an der Dotierungstelle die Entwicklung einer blauen Färbung zu beobachten.

Im Anschluss daran werden die angefärbten Stellen mit einem Skalpell geringfügig ausgeschnitten und anschließend mit Elmex-Gelee (Fa. Wybert) befüllt.

### Anwendungsbeispiel 5

### Bestimmung der Milchsäurebildung über eine Alginatabformmasse auf Zähnen

Zu 20 g Alginat wurden 40 ml Lösung, enthaltend 0,26 g Glycylglycin, 0,24 g Tri-(hydroxymethyl)aminomethan, 36 mg NAD, 0,9 mg Phenazinmethosulfat, 3 mg 3-(4,5-Dimethylthiazolyl-2)-2,5-diphenyltetrazoliumbromid (MTT), 1850 Units Lactatdehydrogenase aus Schweineherz gegeben und mit einem breitem Spatel innerhalb 1 min zu einer homogenen Masse geknetet. Die Alginatmasse wurde in einen handelsüblichen Abformlöffel eingebracht und am Ober- oder Unterkiefer eines Patienten plaziert. Der Patient sollte zuvor die Zähne geputzt und mit einer 1 %igen Saccharose-Lösung gespült haben. Nach 4 min wurde der Abformlöffel entnommen. Stellen mit Milchsäurebildung sind an der entstehenden blauen Färbung zu erkennen.

Im Anschluss daran werden die angefärbten Stellen mit einem Skalpell geringfügig ausgeschnitten und anschließend mit Elmex-Gelee (Fa. Wybert) befüllt. Die so präparierte ausgehärtete Abformmasse wird nun im Munde des Patienten reponiert und dort für einen Zeitraum von ca. 10 Minuten belassen.

## Patentansprüche

1. Verwendung einer Abformmasse, enthaltend diagnostische Zusätze, die bei Kontakt mit infiziertem Gewebe ein identifizierbares Signal erzeugen, zur Herstellung einer Negativform auf die ein Behandlungsmittel aufgebracht wird, zur ortsspezifischen Behandlung von infiziertem Hartgewebe an den Stellen, die als infiziert detektiert wurden.

2. Verwendung nach Anspruch 1, wobei die Abformmasse gewählt ist aus:
(i) Abformmassen oder Filme auf Silikon-, Polyethersilikon-, Polyether-, Alginat- oder Hydrokolloidbasis,
(ii) Kunststoffe aus der Gruppe Polyethylene, Polypropylene, Poly(meth)acrylate, Polyurethane, Polycarbonate, Polysulfid, Polyvinylchloride oder Kautschuk,
(iii) Hydrogele auf Polyvinylpyrrolidon- oder Polyvinylalkoholbasis, oder
(iv) dentale Gipszubereitungen.

3. Verwendung nach einem der vorstehenden Ansprüche, wobei die diagnostischen Zusätze in einer Menge von 0,0001 bis 10 Gew.-%, bevorzugt 0,01 bis 1 Gew.-% vorhanden sind.

4. Verwendung nach einem der vorstehenden Ansprüche, wobei die diagnostischen Zusätze in mikroverkapselter Form vorliegen.

5. Verwendung nach einem der vorstehenden Ansprüche, wobei die diagnostischen Zusätze gewählt sind aus: Farbstoffindikatoren, Fluoreszenzindikatoren, Chemolumineszenzindikatoren, Vitalitätsindikatoren, Antikörpern, Enzymen, Puffersubstanzen und weiteren Hilfsstoffen.

6. Verwendung nach einem der Ansprüche 1 bis 3, wobei das Behandlungsmittel gewählt ist aus: Fluoridierungsmitteln, Antibiotika, Bakteriostatika, quartäre Ammoniumverbindungen und Mineralienmischungen, welche eine effiziente Remineralisierung der Zahnhartsubstanz ermöglichen.

7. Verwendung nach einem der vorstehenden Ansprüche, wobei die Oberfläche der Negativform an den Stellen an denen das Behandlungsmittel aufgebracht werden soll vorbehandelt ist.

8. Behandlungsvorrichtung, erhältlich durch a) Bereitstellen einer Abformmasse, enthaltend diagnostische Zusätze, die bei Kontakt mit infiziertem Gewebe ein identifizierbares Signal erzeugen, b) Abformen von Hartgewebe unter Bildung eines Negativabdrucks, c) Aufbringen eines Behandlungsmittels an den Stellen des Negativabdrucks, an denen ein identifizierbares Signal detektierbar ist.

9. Kit, umfassend ein Trägermaterial oder zumindest eine Komponente eines Trägermaterials, diagnostische Zusätze und ein Behandlungsmittel gewählt aus Fluoridierungsmitteln, Antibiotika, Bakteriostatika, quartäre Ammoniumverbindungen und Mineralienmischungen, welche eine effiziente Remineralisierung der Zahnhartsubstanz ermöglichen.

10. Verfahren zur Herstellung einer individuellen Behandlungsvorrichtung, umfassend den Schritt: Auftragen eines Behandlungsmittels an den Stellen einer Negativfom, die unter Verwendung einer Abformmasse, enthaltend diagnostische Zusätze, die bei Kontakt mit infiziertem Gewebe ein identifizierbares Signal erzeugen, gefertigt wurde, an denen ein identifizierbares Signal detektierbar ist.

11. Verfahren zur Herstellung einer individuellen Behandlungsvorrichtung, umfassend die Schritte a) Anfertigen einer Positivform unter Verwendung der in Anspruch 10 beschriebenen Negativform, b) Anfertigen einer Bissschiene unter Verwendung der Positivform aus Schritt a), c) Übertragen oder Markieren der Stellen von der Negativform, an denen ein identifizierbares Signal detektierbar ist auf die Bissschiene.

12. Verfahren nach Anspruch 11, umfassend Schritt d): Aufbringen eines Behandlungsmittels an den markierten Stellen gemäß Schritt c).

## Claims

1. Use of an impression material, which contains diagnostic additives which generate an identifiable signal on contact with infected tissue, for preparing a negative mold to which a treatment agent is applied for treating infected hard tissue, in a site-specific manner, at the sites which have been found by detection to be infected.

2. Use according to claim 1, where the impression material is selected from:
(i) impression materials or films based on silicone, polyether silicone, polyether, alginate or hydrocolloid,
(ii) synthetic materials derived from the group polyethylenes, polypropylenes, poly(meth)acrylates, polyurethanes, polycarbonates, polysulfide, polyvinyl chlorides or rubber,
(iii) hydrogels based on polyvinylpyrrolidone or polyvinyl alcohol, or
(iv) dental gypsum preparations.

3. Use according to one of the preceding claims, where the diagnostic additives are present in a quantity of from 0.0001 to 10% by weight, preferably of from 0.01 to 1% by weight.

4. Use according to one of the preceding claims, where the diagnostic additives are present in microencapsulated form.

5. Use according to one of the preceding claims, where the diagnostic additives are selected from: dye indicators, fluorescent indicators, chemoluminescent indicators, vital indicators, antibodies, enzymes, buffering substances and other auxiliary substances.

6. Use according to one of Claims 1 to 3, where the treatment agent is selected from: fluoridating agents, antibiotics, bacteriostatics, quaternary ammonium compounds and mineral mixtures which enable the hard tooth substance to be remineralized efficiently.

7. Use according to one of the preceding claims, where the surface of the negative mold is pretreated at the sites to which the treatment agent is to be applied.

8. Treatment device which can be obtained by a) providing an impression material which contains diagnostic additives which generate an identifiable signal on contact with infected tissue, b) molding hard tissue with the formation of a negative imprint, and c) applying a treatment agent to the sites on the negative imprint at which an identifiable signal can be detected.

9. Kit, which comprises a support material, or at least one component of a support material, diagnostic additives and a treatment agent selected from fluoridating agents, antibiotics, bacteriostatics, quaternary ammonium compounds and mineral mixtures which enable the hard tooth substance to be remineralized efficiently.

10. Method for producing an individual treatment device, comprising the step of: applying a treatment agent to the sites, at which an identifiable signal can be detected, on a negative mold which was made using an impression material which contains diagnostic additives which generate an identifiable signal on contact with infected tissue.

11. Method for producing an individual treatment device, comprising the steps of a) making a positive mold using the negative mold described in Claim 10, b) making a mouthpiece using the positive mold from step a), and c) transferring to, or marking on, the mouthpiece the sites on the negative mold at which an identifiable signal can be detected.

12. Method according to Claim 11, comprising step d): applying a treatment agent to the sites which were labeled as described in step c).

## Revendications

1. Utilisation d'une pâte de moulage d'empreintes qui contient des additifs de diagnostic qui créent un signal identifiable lorsqu'ils entrent en contact avec un tissu infecté, en vue de la préparation d'une forme négative sur laquelle un agent de traitement est appliqué en vue du traitement local spécifique d'un tissu dur infecté aux emplacements sur lesquels on a détecté l'infection.

2. Utilisation selon la revendication 1, dans laquelle la pâte de moulage d'empreintes est sélectionnée parmi :
(i) les pâtes de moulage d'empreintes ou films à base de silicone, de polyéther-silicone, de polyéther, d'alginate ou d'hydrocolloïdes,
(ii) les matières synthétiques sélectionnées dans le groupe constitué des polyéthylènes, des polypropylènes, des poly(méthacrylate), des polyuréthanes, des polycarbonates, des polysulfures, des poly(chlorure de vinyle) ou du caoutchouc,
(iii) des hydrogels à base de polyvinylpyrrolidone ou d'alcool polyvinylique ou
(iv) des préparations de plâtre dentaire.

3. Utilisation selon l'une des revendications précédentes, dans laquelle les additifs de diagnostic sont présents en quantité de 0,0001 à 10 % en poids et de préférence de 0,01 à 1 % en poids.

4. Utilisation selon l'une des revendications précédentes, dans laquelle les additifs de diagnostic sont présents sous forme microencapsulée.

5. Utilisation selon l'une des revendications précédentes, dans laquelle les additifs de diagnostic sont sélectionnés parmi les indicateurs colorés, les indicateurs par fluorescence, les indicateurs par chimioluminescence, les indicateurs de vitalité, les anticorps, les enzymes, les substances tampons et d'autres substances auxiliaires.

6. Utilisation selon l'une des revendications 1 à 3, dans laquelle l'agent de traitement est sélectionné parmi les agents de fluoration, les antibiotiques, les bactériostatiques, les composés d'ammonium quaternaire et les mélanges de minéraux qui permettent une reminéralisation efficace de la substance dure de la dent.

7. Utilisation selon l'une des revendications, dans laquelle la surface de la forme négative est prétraitée aux emplacements sur lesquels l'agent de traitement doit être appliqué.

8. Dispositif de traitement obtenu en a) préparant une pâte de moulage d'empreintes qui contient des additifs de diagnostic qui créent un signal identifiable lorsqu'ils sont mis en contact avec le tissu infecté, b) moulage de l'empreinte du tissu dur pour former une empreinte négative et c) appliquant un agent de traitement aux emplacements de l'empreinte négative sur lesquels le signal identifiable a été détecté.

9. Trousse qui comprend un matériau de support ou au moins un composant d'un matériau de support, des additifs de diagnostic et un agent de traitement sélectionné parmi les agents de fluoration, les antibiotiques, les bactériostatiques, les composés d'ammonium quaternaire et les mélanges de minéraux qui permettent une reminéralisation efficace de la substance dure de la dent.

10. Procédé de préparation d'un dispositif de traitement individualisé, qui comprend l'étape qui consiste à appliquer un agent de traitement aux emplacements d'une forme négative qui a été préparée en recourant à une pâte de moulage d'empreintes qui comprend des additifs de diagnostic qui créent un signal identifiable lorsqu'ils sont mis en contact avec le tissu infecté, et sur lesquels un signal identifiable peut être détecté.

11. Procédé de préparation d'un dispositif de traitement individualisé, qui comprend les étapes qui consistent à a) préparer une forme positive en recourant à la forme négative décrite dans la revendication 10, b) préparer un rail dentaire en recourant à la forme positive de l'étape a) et c) transférer ou marquer les emplacements de la forme négative auxquels un signal identifiable a été détecté sur le rail dentaire.

12. Procédé selon la revendication 11, qui comprend l'étape d) qui consiste à appliquer un agent de traitement sur les emplacements marqués de l'étape c).
